# EUROPÄISCHE PATENTSCHRIFT

(11) **EP 0 667 155 B1**
(45) Veröffentlichungstag und Bekanntmachung des Hinweises auf die Patenterteilung: **05.04.2000**
(21) Anmeldenummer: 94120500.7
(22) Anmeldetag: 23.12.1994
(51) Int. Cl.: A61K 31/385

(54) **Tabletten mit Thioctsäure bestimmter Korngrösse**
Tablets containing thioctic acid with a specified size
Comprimés contenant de l'acide thioctique de taille bien définie

(30) Priorität: 10.01.1994 DE 4400269
(43) Veröffentlichungstag der Anmeldung: 16.08.1995
(73) Patentinhaber: ASTA Medica Aktiengesellschaft, 01277 Dresden (DE)
(72) Erfinder: Sarlikiotis, Werner, Dr., D-60316 Frankfurt/M. (DE); Hettche, Helmut, Dr., D-63128 Dietzenbach-Steinberg (DE)
(74) Vertreter: Nauwald, Gunter

(56) Entgegenhaltungen:
- EP-A- 0 560 092
- SUCKER ET AL 'PHARMAZEUTISCHE TECHNOLOGIE' 1991 , THIEME VERLAG , STUTTGART DE XP 000189560 * Seite 157 - Seite 160 * * Seite 157; Tabelle 4.7 *
- ROTH H.J. ET AL 'HAGERS HANDBUCH DER PHARMAZEUTISCHEN PRAXIS ' 1971 , SPRINGER VERLAG , BERLIN-HEIDELBERG-NEW YORK * Seite 708 - Seite 717 * * Seite 715 - Seite 716; Tabellen *

## Beschreibung

Die vorliegende Erfindung betrifft eine Arzneimittelformulierung in Tablettenform enthaltend Thioctsäure bestimmter Korngröße sowie gegebenenfalls pharmazeutisch unbedenkliche Hilfsstoffe.

Thioctsäure (α-Liponsäure) ist chemisch eine 1, 2- Di- thiacyclopentan-3-valeriansäure. Die Erfindung bezieht sich nicht nur auf die razemische Form, sondern ebenso auf die reine (R)- beziehungsweise (S)-Thioctsäure sowie auf Gemische aus (R)- und (S)-Thioctsäure mit beliebiger Zusammensetzung. Thioctsäure ist Bestandteil des Zellstoffwechsels und wird daher in vielen Pflanzen und tierischen Organismen gefunden. Sie wirkt als eines der Coenzyme bei der oxydativen Decarboxylierung von Pyruvat und anderen α-Ketosäuren. Thioctsäure wird seit längerer Zeit bei verschiedenen Erkrankungen eingesetzt, so unter anderem bei Lebererkrankungen, bei Leberschädigungen durch Pilzvergiftung sowie bei diabetischer und alkoholischer Polyneuropathie, einer mit Stoffwechselerkrankungen einhergehenden Veränderung peripherer Nerven.

Zur Erleichterung der Einnahme und zur Erhöhung der Akzeptanz beim Patienten besteht der Bedarf für höher konzentrierte Thioctsäure-Tabletten mit kleinerem Format.
Die proportionale Vervielfachung der Bestandteile in der 200 mg Thioctsäure enthaltenden Tablette führt bei Wirkstoffdosierungen von mehr als 500 mg pro Tablette bereits zu Tabletten mit Einzelgewichten von mehr als 1,2 g. Tabletten mit solch hohem Einzelgewicht sind aufgrund ihrer Größe nur schwer schluckbar und führen zu einer Verschlechterung der Akzeptanz. Es ist erforderlich, den Hilfsstoffanteil in den höher dosierten festen Arzneiformen zu reduzieren. Nun lassen sich jedoch thioctsäurehaltige Tabletten mit reduziertem Hilfsstoffanteil mit üblichen Herstellungsverfahren nicht in befriedigender Qualität herstellen. Höhere Konzentrationen von Thioctsäure führen zu Problemen bei der Tablettenpressung. Die Preßmassen neigen zur Anhaftung an den Preßwerkzeugen (siehe auch Hagers Handbuch der pharmazeutischen Praxis, Seiten 708 bis 717, Springer Verlag, Berlin-Heidelberg-New York 1971).
Darüberhinaus treten bei den Tabletten Risse parallel zur Oberfläche auf, bei bikonvexen Tabletten kommt es zum Absprengen der Kugelkappen (Deckeln). Diese Störungen sind bedingt durch die Eigenschaften der Thioctsäure; als besonders kritisch erweist sich der niedrige Schmelzpunkt der Substanz von 60, 5°C (R, S-Thioctsäure) beziehungsweise 48,3°C (R-Thioctsäure) und 48,5°C (S-Thioctsäure).

Durch Reduktion des Anteils der Hilfsstoffe in der Tablette reduziert man gleichzeitig auch die Wahrscheinlichkeit von Unverträglichkeitsreaktionen, wie zum Beispiel auf Lactose (Deutsche Apothekerzeitung 131, 1569 (1991)).

Eine bereits beschriebene Möglichkeit zur Lösung des Problems besteht darin, den Wirkstoff Thioctsäure mit viel Wasser zu granulieren (siehe EP 0 560 092 A1). Nachteilig an dieser Arbeitsweise ist, daß die Gefahr der Überfeuchtung der zu granulierenden Masse besteht, sodaß eine anschließende Trocknung nur noch mit Mühe durchführbar ist, da eine halbflüssige Masse entstehen kann. Auch ist generell die Trocknung von Granulaten mit hohem Flüssigkeitsanteil aufwendig, kostenintensiv und langwierig. Weiterhin ist aus Sucker et al., Pharmazeutische Technologie, Seiten 157 bis 160, Thieme Verlag, Stuttgart 1991, bekannt, daß eine kleine Teilchengröße des Wirkstoffs zu einer vermehrten Wasseradsorption, und damit zu einer verringerten mechanischen Widerstansfähigkeit von aus dem Wirkstoff gepreßten Tabletten, führt.

Nun wurde überraschend gefunden, daß durch den Einsatz von Thioctsäure bestimmter Korngröße sich die Verpreßbarkeit der Thioctsäure zu Tabletten wesentlich verbessern läßt. So ist es vorteilhaft, daß 20 % bis 100 % der eingesetzten Thioctsäure eine Partikelgröße größer als 100 µm aufweisen, insbesondere daß 30 % bis 80 % der Wirkstoffpartikel größer als 200 µm sind. Idealerweise sollten 20 % bis 100 % der eingesetzten Thioctsäure aus Partikeln zwischen 100 µm und 710 µm und insbesondere 30 % bis 80 % aus Partikeln zwischen 200 µm und 710 µm bestehen. Der Einsatz feinkristalliner Thioctsäure mit Korngröße 90 % < 50 µm führt bei der Tablettierung zu unbefriedigenden Ergebnissen. Da feinkörnige Thioctsäure zur Agglomeration neigt, eignet sich die Siebanalyse nicht, um die Größenverteilung der Wirkstoffpartikel zu bestimmen. Gegeigneter dafür ist die Laserbeugungsspektrometrie. Für größere Wirkstoff eigen sich jedoch beide Methoden. Bei dem daraus erhaltenen Ergebnissen handelt es sich um Volumen - und damit um Massenverteilungen.

Um die erfindungsgemäßen Tabletten herzustellen, werden bekannte Verfahren eingesetzt. So kann der Wirkstoff allein oder als Gemisch mit Hilfsstoffen mit einer Flüssigkeit angefeuchtet werden. Falls erforderlich, wird anschließend die feuchte Masse durch ein Passiergerät mit einer Ringmatritze (zum Beispiel Alexanderwerk - Reibschnitzler, Stephan-Granuliermaschine) oder ein Granuliersieb gegeben.
Die weiteren Schritte der Bearbeitung werden entsprechend dem Stand der Technik durchgeführt, so beispielsweise Trocknung, gegebenenfalls Siebung, Mischung mit weiteren Hilfsstoffen und Verpressung zu Tabletten.
Die entsprechend der genannten Arbeitsweise hergestellten Mischungen lassen sich einwandfrei zu Tabletten verpressen, es kommt nicht zu einer Anhaftung an den Preßwerkzeugen.

Als Flüssigkeiten zur Granulation können eingesetzt werden: Alkohole mit 1 - 4 C-Atomen, Ester aus niederen organischen Säuren und niederen organischen Alkoholen mit insgesamt bis zu 6 C-Atomen, zum Beispiel Methanol, Ethanol, Isopropanol, Essigsäureethylester und besonders bevorzugt Wasser. Auch Lösungen von Bindemitteln können Einsatz bei der Granulation finden.
So können als Bindemittel für diesen Zweck alle pharmazeutisch gebräuchlichen Bindemittel wie Cellulosederivate (zum Beispiel Ethylcellulose, Methylcellulose, Hydroxyethylcellulose, Carboxymethylcellulose, Hydroxypropylmethylcellulose), Gelatine, Stärke, Polyglycole (mittlere Molmasse 1000 - 35000 Dalton), Polyvinylalkohole, Polyvinylpyrrolidon, Polyacrylsäure, Vinylpyrrolidon-Vinylacetat- Copolymerisat, Alginate, Saccharose oder Glucose, Polysaccharide, wie zum Beispiel natürliche Gummisorten, wie zum Beispiel Gummi Arabicum, Tragant, Pektin, Guar-Gummi in Mengen von 1 - 30 Gewichts%, vorzugsweise 5 - 20 Gewichts%, insbesondere 10 - 15 Gewichts% bezogen auf den Wirkstoff, eingesetzt werden (Konzentration der wässrigen Bindemittellösungen 2 - 30 Gewichts% vorzugsweise 5 - 15 % Gewichts%).
Dabei können auch gleichzeitig verschiedene Bindemittel, zum Beispiel unterschiedliche Cellulosederivate nebeneinander, eingesetzt werden. Die Bindemittel können in die trockene Pulvermischung eingearbeitet werden oder in der Granulierflüssigkeit gelöst oder dispergiert eingebracht werden. Auch die Kombination von trocken vorgelegtem und gelöstem beziehungsweise dispergiertem Bindemittel ist geeignet.

Neben der Thioctsäure kann das Granulat noch gebräuchliche Tablettenhilfsstoffe wie Füll-, Spreng- und Netzmittel enthalten. Die Tablette kann neben dem Granulat noch weitere Füll-, Binde-, Spreng-, Netz-, Fließhilfsmittel, Schmiermittel und Gegenklebemittel enthalten.
Auch ist es möglich, die Thioctsäure in nicht granulierter Form direkt einzusetzen.
Es wird, falls erforderlich, das Granulat oder der Wirkstoff mit Füll-, Binde-, Spreng-, Netz-, Fließhilfsmittel, Schmiermittel und/oder Gegenklebemittel versetzt.
Als Füllmittel können beispielsweise eingesetzt werden: Cellulose, Cellulosederivate, Saccharose, Lactose, Glucose, Fructose, Calciumphosphate, Calciumsulfate, Calciumcarbonate, Stärke, modifizierte Stärke, Zuckeralkohole wie Sorbitol oder Mannitol.
Als Bindemittel eignen sich die bereits oben genannten Bindemittel.
Als Sprengmittel können beispielsweise eingesetzt werden: Stärke, modifizierte Stärke (beispielsweise Natrium-Stärkeglycolat, Starch 1500), Cellulose, Cellulosederivate, Alginate, quervernetztes Polyvinylpyrrolidon oder quervernetzte Carboxymethylcellulose (Ac-Di-Sol/FMC).
Als Netzmittel sind beispielsweise einsetzbar: Natriumdioctylslfosuccinat, Natriumlaurylsulfat, Polysorbate oder Polyoxyethylen-Stearinsäureester. Als Fließhilfsmittel eignen sich beispielsweise:
Kolloidales Siliciumdioxid, Talkum oder Magnesiumstearat.

Als Schmiermittel können beispielsweise eingesetzt werden: Magnesiumstearat, Calciumstearat, D, L-Leucin, Talkum, Stearinsäure, Polyglycole (mittlere Molmasse 3000 - 35000), Fettalkohole oder Wachse.
Als Gegenklebemittel sind beispielsweise einsetzbar: Stärke, Talkum, Magnesiumstearat, Calciumstearat oder D, L- Leucin.

Die so hergestellte Mischung wird in üblicher Weise zu Tabletten verpreßt. Dabei kann es von Vorteil sein, die Preßmassentemperatur vor der Verpressung unter die Raumtemperatur abzusenken. Die Preßmassentemperatur kann 0°C - 30°C, vorzugsweise 5°C - 20°C, insbesondere 8°C - 15°C betragen.

Die erfindungsgemäßen Arzneimittelformulierungen enthalten zwischen 45 Gewichts% und 99,9 Gewichts% Wirkstoff, bevorzugt zwischen 55 Gewichts% und 99,9 Gewichts%, insbesondere zwischen 73 Gewichts% und 99,9 Gewichts% und besonders bevorzugt zwischen 80 Gewichts% und 99,9 Gewichts% Wirkstoff.

Die Tabletten können nach üblichen Methoden mit einem magensaft- permeablen oder magensaftlöslichen Überzug versehen werden.

### Beispiel 1:

### Tabletten mit 600 mg Thioctsäure auf 684 mg Tablettengewicht

### (Granulationsverfahren)

1200 g Thioctsäure mit Partikelgröße 60 % > 100 µm werden mit 120 g Hydroxypropylcellulose, niedrig substituiert (L-HPC-LH 22/Shin Etsu) gemischt und die Mischung mit 600 g gereinigtem Wasser befeuchtet und durchgearbeitet. Nach Passage durch ein Sieb der Maschenweite 2 mm wird das Granulat getrocknet, nochmals durch ein Sieb der Maschenweite 1 mm gesiebt und nach Zumischen von 48 g Magnesiumstearat zu Tabletten in Oblong-Format vom Gewicht 684 mg, einer Länge von 18 mm, einer Breite von 8 mm und einem Wölbungsradius von 6 mm gepreßt. Eine Tablette enthält 600 mg Thioctsäure. Das Tablettieren bereitet keine Probleme. Die Tabletten können anschließend nach üblichen Standardverfahren mit einem magensaftlöslichen oder magensaftpermeablen Filmüberzug versehen werden.

### Beispiel 2

### Tabletten mit 600 mg Thioctsäure auf 684 mg Tablettengewicht

### (Granulationsverfahren)

Folgendes Beispiel soll die Probleme aufzeigen, welche auftreten, wenn feinkörniger Wirkstoff eingesetzt wird. Die Zusammensetzung und das Herstellungsverfahren sind dem Beispiel 1 gleich.

1200 g Thioctsäure mit Partikelgröße 90 % < 50 µm werden mit 120 g Hydroxypropylcellulose, niedrig substituiert (L-HPC-LH 22/Shin Etsu) gemischt und die Mischung mit 600 g gereinigtem Wasser befeuchtet und durchgearbeitet. Nach Passage durch ein Sieb der Maschenweite 2 mm wird das Granulat getrocknet, nochmals durch ein Sieb der Maschenweite 1 mm gesiebt und nach Zumischen von 48 g Magnesiumstearat zu Tabletten im Oblong-Format vom Gewicht 684 mg, einer Länge von 18 mm, einer Breite von 8 mm und einem Wölbungsradius von 6 mm gepreßt. Eine Tablette enthält 600 mg Thioctsäure. Nach kurzer Zeit tritt großflächiges Anhaften der Preßmasse an die Tablettierstempel auf. Da dieser Effekt durch Änderung der Einstellungen der Tablettenpresse nicht zu beheben ist, muß der Preßvorgang abgebrochen werden.

### Beispiel 3

### Tabletten mit 200 mg Thioctsäure auf 235 mg Tablettengewicht

### (Direktpreßmasse)

2100,00 g erfindungsgemäße Thioctsäure werden mit 113,75 g Lactose, 175,00 g mikrokristalliner Cellulose, 17,50 g Polyvidon, 29,75 g Maisstärke und 1,75 g hochdispersem Siliciumoxid gemischt. Anschließend werden zu dieser Mischung 29,75 g Magnesiumstearat zugegeben und erneut gemischt. Die so hergestellte Preßmasse wird zu Tabletten mit einem Gewicht von 235 mg verpreßt.
Die Tabletten enthalten 200 mg Thioctsäure, das entspricht einem Wirkstoffgehalt von 85,1 %

## Patentansprüche

1. Arzneimittelformulierung in Form von Tabletten enthaltend Thioctsäure (α-Liponsäure) mit einem Gehalt zwischen 45 Gew. % und 99,0 Gew. %, dadurch gekennzeichnet, daß 20% bis 100% des eingesetzten Wirkstoffes eine Partikelgröße größer als 100 µm aufweisen.

2. Arzneimittelformulierung in Form von Tabletten enthaltend Thioctsäure (α-Liponsäure) mit einem Gehalt zwischen 55 Gew. % und 99,9 Gew. % gemäß Anspruch 1, dadurch gekennzeichnet, daß 20% bis 100% des eingesetzten Wirkstoffes eine Partikelgröße größer als 100 µm aufweisen.

3. Arzneimittelformulierung in Form von Tabletten enthaltend Thioctsäure (α-Liponsäure) mit einem Gehalt zwischen 73 Gew. % und 99,9 Gew. % gemäß Anspruch 1, dadurch gekennzeichnet, daß 20% bis 100% des eingesetzten Wirkstoffes eine Partikelgröße größer als 100 µm aufweisen.

4. Arzneimittelformulierung in Form von Tabletten enthaltend Thioctsäure (α-Liponsäure) mit einem Gehalt zwischen 80 Gew. % und 99,9 Gew. % gemäß Anspruch 1, dadurch gekennzeichnet, daß 20% bis 100% des eingesetzten Wirkstoffes eine Partikelgröße von größer als 100 µm aufweisen.

5. Arzneimittelformulierung in Form von Tabletten enthaltend Thioctsäure (α-Liponsäure) mit einem Gehalt zwischen 45 Gew. % und 99,9 Gew. % gemäß Anspruch 1, dadurch gekennzeichnet, daß 20% bis 100% des eingesetzten Wirkstoffes eine Partikelgröße von 100 µm bis 710 µm aufweisen.

6. Arzneimittelformulierung in Form von Tabletten enthaltend Thioctsäure (α-Liponsäure) mit einem Gehalt zwischen 55 Gew. % und 99,9 Gew. % gemäß Anspruch 1 dadurch gekennzeichnet, daß 20% bis 100% des eingesetzten Wirkstoffes eine Partikelgröße von 100 µm bis 710 µm aufweisen.

7. Arzneimittelformulierung in Form von Tabletten enthaltend Thioctsäure (α-Liponsäure) mit einem Gehalt zwischen 73 Gew. % und 99,9 Gew. % gemäß Anspruch 1, dadurch gekennzeichnet, daß 20% bis 100% des eingesetzten Wirkstoffes eine Partikelgröße von 100 µm bis 710 µm aufweisen.

8. Arzneimittelformulierung in Form von Tabletten enthaltend Thioctsäure (α-Liponsäure) mit einem Gehalt zwischen 80 Gew. % und 99,9 Gew. % gemäß Anspruch 1, dadurch gekennzeichnet, daß 20% bis 100% des eingesetzten Wirkstoffes eine Partikelgröße von 100 µm bis 710 µm aufweisen.

9. Arzneimittelformulierung in Form von Tabletten enthaltend Thioctsäure (α-Liponsäure) mit einem Gehalt zwischen 45 Gew. % und 99,9 Gew. % gemäß Anspruch 1, dadurch gekennzeichnet, daß 30% bis 80% des eingesetzten Wirkstoffes eine Partikelgröße von größer als 200 µm aufweisen.

10. Arzneimittelformulierung in Form von Tabletten enthaltend Thioctsäure (α-Liponsäure) mit einem Gehalt zwischen 55 Gew. % und 99,9 Gew. % gemäß Anspruch 1, dadurch gekennzeichnet, daß 30% bis 80% des eingesetzten Wirkstoffes eine Partikelgröße von größer als 200 µm aufweisen.

11. Arzneimittelformulierung in Form von Tabletten enthaltend Thioctsäure (α-Liponsäure) mit einem Gehalt zwischen 73 Gew. % und 99,9 Gew. % gemäß Anspruch 1, dadurch gekennzeichnet, daß 30% bis 80% des eingesetzten Wirkstoffes eine Partikelgröße von größer als 200 µm aufweisen.

12. Arzneimittelformulierung in Form von Tabletten enthaltend Thioctsäure (α-Liponsäure) mit einem Gehalt zwischen 80 Gew. % und 99,9 Gew. % gemäß Anspruch 1, dadurch gekennzeichnet, daß 30% bis 80% des eingesetzten Wirkstoffes eine Partikelgröße von größer als 200 µm aufweisen.

13. Arzneimittelformulierung in Form von Tabletten enthaltend Thioctsäure (α-Liponsäure) mit einem Gehalt zwischen 45 Gew. % und 99,9 Gew. % gemäß Anspruch 1, dadurch gekennzeichnet, daß 30% bis 80% des eingesetzten Wirkstoffes eine Partikelgröße von 200 µm bis 710 µm aufweisen.

14. Arzneimittelformulierung in Form von Tabletten enthaltend Thioctsäure (α-Liponsäure) mit einem Gehalt zwischen 55 Gew. % und 99,9 Gew. % gemäß Anspruch 1, dadurch gekennzeichnet, daß 30% bis 80% des eingesetzten Wirkstoffes eine Partikelgröße von 200 µm bis 710 µm aufweisen.

15. Arzneimittelformulierung in Form von Tabletten enthaltend Thioctsäure (α-Liponsäure) mit einem Gehalt zwischen 73 Gew. % und 99,9 Gew. % gemäß Anspruch 1, dadurch gekennzeichnet, daß 30% bis 80% des eingesetzten Wirkstoffes eine Partikelgröße von 200 µm bis 710 µm aufweisen.

16. Arzneimittelformulierung in Form von Tabletten enthaltend Thioctsäure (α-Liponsäure) mit einem Gehalt zwischen 80 Gew. % und 99,9 Gew. % gemäß Anspruch 1, dadurch gekennzeichnet, daß 30% bis 80% des eingesetzten Wirkstoffes eine Partikelgröße von 200 µm bis 710 µm aufweisen.

17. Arzneimittelformulierung gemäß den Ansprüchen 1 bis 16, dadurch gekennzeichnet, daß neben Thioctsäure weitere physiologisch akzeptable Hilfsstoffe enthalten sind.

18. Partikel, bestehend aus Thioctsäure (α-Liponsäure), dadurch gekennzeichnet, daß 20% bis 100% der Partikel eine Partikelgröße größer als 100 µm aufweisen.

## Claims

1. Pharmaceutical formulation in the form of tablets comprising thioctic acid (α-lipoic acid) having a content of between 45% and 99.0% by weight, characterized in that 20% to 100% of the active compound employed have a particle size of greater than 100 µm.

2. Pharmaceutical formulation in the form of tablets comprising thioctic acid (α-lipoic acid) having a content of between 55% and 99.9% by weight according to Claim 1, characterized in that 20% to 100% of the active compound employed have a particle size of greater than 100 µm.

3. Pharmaceutical formulation in the form of tablets comprising thioctic acid (α-lipoic acid) having a content of between 73% and 99.9% by weight according to Claim 1, characterized in that 20% to 100% of the active compound employed have a particle size of greater than 100 µm.

4. Pharmaceutical formulation in the form of tablets comprising thioctic acid (α-lipoic acid) having a content of between 80% and 99.9% by weight according to Claim 1, characterized in that 20% to 100% of the active compound employed have a particle size of greater than 100 µm.

5. Pharmaceutical formulation in the form of tablets comprising thioctic acid (α-lipoic acid) having a content of between 45% and 99.9% by weight according to Claim 1, characterized in that 20% to 100% of the active compound employed have a particle size from 100 µm to 710 µm.

6. Pharmaceutical formulation in the form of tablets comprising thioctic acid (α-lipoic acid) having a content of between 55% and 99.9% by weight according to Claim 1, characterized in that 20% to 100% of the active compound employed have a particle size from 100 µm to 710 µm.

7. Pharmaceutical formulation in the form of tablets comprising thioctic acid (α-lipoic acid) having a content of between 73% and 99.9% by weight according to Claim 1, characterized in that 20% to 100% of the active compound employed have a particle size from 100 µm to 710 µm.

8. Pharmaceutical formulation in the form of tablets comprising thioctic acid (α-lipoic acid) having a content of between 80% and 99.9% by weight according to Claim 1, characterized in that 20% to 100% of the active compound employed have a particle size from 100 µm to 710 µm.

9. Pharmaceutical formulation in the form of tablets comprising thioctic acid (α-lipoic acid) having a content of between 45% and 99.9% by weight according to Claim 1, characterized in that 30% to 80% of the active compound employed have a particle size of greater than 200 µm.

10. Pharmaceutical formulation in the form of tablets comprising thioctic acid (α-lipoic acid) having a content of between 55% and 99.9% by weight according to Claim 1, characterized in that 30% to 80% of the active compound employed have a particle size of greater than 200 µm.

11. Pharmaceutical formulation in the form of tablets comprising thioctic acid (α-lipoic acid) having a content of between 73% and 99.9% by weight according to Claim 1, characterized in that 30% to 80% of the active compound employed have a particle size of greater than 200 µm.

12. Pharmaceutical formulation in the form of tablets comprising thioctic acid (α-lipoic acid) having a content of between 80% and 99.9% by weight according to Claim 1, characterized in that 30% to 80% of the active compound employed have a particle size of greater than 200 µm.

13. Pharmaceutical formulation in the form of tablets comprising thioctic acid (α-lipoic acid) having a content of between 45% and 99.9% by weight according to Claim 1, characterized in that 30% to 80% of the active compound employed have a particle size from 200 µm to 710 µm.

14. Pharmaceutical formulation in the form of tablets comprising thioctic acid (α-lipoic acid) having a content of between 55% and 99.9% by weight according to Claim 1, characterized in that 30% to 80% of the active compound employed have a particle size from 200 µm to 710 µm.

15. Pharmaceutical formulation in the form of tablets comprising thioctic acid (α-lipoic acid) having a content of between 73% and 99.9% by weight according to Claim 1, characterized in that 30% to 80% of the active compound employed have a particle size from 200 µm to 710 µm.

16. Pharmaceutical formulation in the form of tablets comprising thioctic acid (α-lipoic acid) having a content of between 80% and 99.9% by weight according to Claim 1, characterized in that 30% to 80% of the active compound employed have a particle size from 200 µm to 710 µm.

17. Pharmaceutical formulation according to Claims 1 to 16, characterized in that further physiologically acceptable auxiliaries are contained in addition to thioctic acid.

18. Particles consisting of thioctic acid (α-lipoic acid), characterized in that 20% to 100% of the particles have a particle size of greater than 100 µm.

## Revendications

1. Formulation de médicament sous forme de comprimés renfermant de l'acide thioctique (acide α-lipoïque) ayant une teneur comprise entre 45 % en poids et 99,0 % en poids,
caractérisée en ce que
de 20 à 100 % du principe actif mis en oeuvre possèdent une taille de particules plus grande que 100 µm.

2. Formulation de médicament sous forme de comprimés renfermant de l'acide thioctique (acide α-lipoïque) ayant une teneur comprise entre 55 % en poids et 99,9 % en poids, conformément à la revendication 1,
caractérisée en ce que
de 20 % à 100 % du principe actif mis en oeuvre possèdent une taille de particules plus grande que 100 µm.

3. Formulation de médicament sous forme de comprimés renfermant de l'acide thioctique (acide α-lipoïque) ayant une teneur comprise entre 73 % en poids et 99,9 % en poids, conformément à la revendication 1,
caractérisée en ce que
de 20 % à 100 % du principe actif mis en oeuvre possèdent une taille de particules plus grande que 100 µm.

4. Formulation de médicament sous forme de comprimés renfermant de l'acide thioctique (acide α-lipoïque) ayant une teneur comprise entre 80 % en poids et 99,9 % en poids, conformément à la revendication 1,
caractérisée en ce que
de 20 % à 100 % du principe actif mis en oeuvre possèdent une taille de particules plus grande que 100 µm.

5. Formulation de médicament sous forme de comprimés renfermant de l'acide thioctique (acide α-lipoïque) ayant une teneur comprise entre 45 % en poids et 99,9 % en poids, conformément à la revendication 1,
caractérisée en ce que
de 20 % à 100 % du principe actif mis en oeuvre possèdent une taille de particules allant de 100 µm à 710 µm.

6. Formulation de médicament sous forme de comprimés renfermant de l'acide thioctique (acide α-lipoïque) ayant une teneur comprise entre 55 % en poids et 99,9 % en poids, conformément à la revendication 1,
caractérisée en ce que
de 20 % à 100 % du principe actif mis en oeuvre possèdent une taille de particules allant de 100 µm à 710 µm..

7. Formulation de médicament sous forme de comprimés renfermant de l'acide thioctique (acide α-lipoïque) ayant une teneur comprise entre 73 % en poids et 99,9 % en poids, conformément à la revendication 1,
caractérisée en ce que
de 20 % à 100 % du principe actif mis en oeuvre possèdent une taille de particules allant de 100 µm à 710 µm.

8. Formulation de médicament sous forme de comprimés renfermant de l'acide thioctique (acide α-lipoïque) ayant une teneur comprise entre 80 % en poids et 99,9 % en poids, conformément à la revendication 1,
caractérisée en ce que
de 20 % à 100 % du principe actif mis en oeuvre possèdent une taille de particules allant de 100 µm à 710 µm.

9. Formulation de médicament sous forme de comprimés renfermant de l'acide thioctique (acide α-lipoïque) ayant une teneur comprise entre 45 % en poids et 99,9 % en poids, conformément à la revendication 1,
caractérisée en ce que
de 30 % à 80 % du principe actif mis en oeuvre possèdent une taille de particules plus grande que 200 µm.

10. Formulation de médicament sous forme de comprimés renfermant de l'acide thioctique (acide α-lipoïque) ayant une teneur comprise entre 55 % en poids et 99,9 % en poids, conformément à la revendication 1,
caractérisée en ce que
de 30 % à 80 % du principe actif mis en oeuvre possèdent une taille de particules de plus de 200 µm.

11. Formulation de médicament sous forme de comprimés renfermant de l'acide thioctique (acide α-lipoïque) ayant une teneur comprise entre 73 % en poids et 99,9 % en poids, conformément à la revendication 1,
caractérisée en ce que
de 30 % à 80 % du principe actif mis en oeuvre possèdent une taille de particules de plus de 200 µm

12. Formulation de médicament sous forme de comprimés renfermant de l'acide thioctique (acide α-lipoïque) ayant une teneur comprise entre 80 % en poids et 99,9 % en poids, conformément à la revendication 1,
caractérisée en ce que
de 30 % à 80 % du principe actif mis en oeuvre possèdent une taille de particules de plus de 200 µm.

13. Formulation de médicament sous forme de comprimés renfermant de l'acide thioctique (acide α-lipoïque) ayant une teneur comprise entre 45 % en poids et 99,9 % en poids, conformément à la revendication 1,
caractérisée en ce que
de 30 % à 80 % du principe actif mis en oeuvre possèdent une taille de particules allant de 200 µm à 710 µm.

14. Formulation de médicament sous forme de comprimés renfermant de l'acide thioctique (acide α-lipoïque) ayant une teneur comprise entre 55 % en poids et 99,9 % en poids, conformément à la revendication 1,
caractérisée en ce que
de 30 % à 80 % du principe actif mis en oeuvre possèdent une taille de particules allant de 200 µm à 710 µm.

15. Formulation de médicament sous forme de comprimés renfermant de l'acide thioctique (acide α-lipoïque) ayant une teneur comprise entre 73 % en poids et 99,9 % en poids, conformément à la revendication 1,
caractérisée en ce que
de 30 % à 80 % du principe actif mis en oeuvre possèdent une taille de particules allant de 200 µm à 710 µm.

16. Formulation de médicament sous forme de comprimés renfermant de l'acide thioctique (acide α-lipoïque) ayant une teneur comprise entre 80 % en poids et 99,9 % en poids, conformément à la revendication 1,
caractérisée en ce que
de 30 % à 80 % du principe actif mis en oeuvre possèdent une taille de particules allant de 200 µm à 710 µm.

17. Formulation de médicament conformément aux revendications 1 à 16,
caractérisée en ce que
à côté de l'acide thioctique d'autres adjuvants physiologiquement acceptables sont contenus.

18. Particule consistant en de l'acide thioctique (acide α-lipoïque),
caractérisée en ce que
de 20 % à 100 % des particules possèdent une taille de particules plus grande que 100 µm.
